# EUROPEAN PATENT SPECIFICATION

(11) **EP 0 651 741 B1**
(45) Date of publication and mention of the grant of the patent: **29.01.1997**
(21) Application number: 93917905.7
(22) Date of filing: 23.07.1993
(51) Int. Cl.: C07C 279/14, A61K 31/195

(54) **NG-MONOMETHYL-L-ARGININE HYDROCHLORIDE DERIVATIVES AND PROCESS FOR THEIR PREPARATION**
NG-MONOMETHYL-L-ARGININ-HYDROCHLORID-DERIVATE UND VERFAHREN ZU DEREN HERSTELLUNG
DERIVES D'HYDROCHLORURE DE NG-MONOMETHYL-L-ARGININE ET LEUR PROCEDE DE PREPARATION

(30) Priority: 24.07.1992 GB 9215816
(43) Date of publication of application: 10.05.1995
(73) Proprietor: THE WELLCOME FOUNDATION LIMITED, London NW1 2BP (GB)
(72) Inventor: HODSON, Harold Francis, Beckenham, Kent BR3 3BS (GB)
(74) Representative: Baker-Munton, Nicola Jane
(86) International application number: GB9301563
(87) International publication number: WO9402453

(56) References cited:
- WO-A-91/04024
- PROC. NATL. ACAD. SCI. USA, vol. 87, May 1990, pages 3629-3632. R. G. KILBOURN et al, "NG-methyl-L-arginine inhibits tumor necrosis factor-induced hypotension: implications for the involvement of nitric oxide"
- CHEMICAL ABSTRACTS, vol. 117, 1992, Columbus, Ohio, US; abstract no. 207812w, page 375, D. A. PETERSON et al, "The nonspecificity of specific nitric oxide synthase inhibitors"

## Description

The present invention relates to novel crystalline salts of (S)-N5-[imino(methyl amino)methyl]ornithine, pharmaceutical compositions containing such salts and their use in medicine, more particularly the treatment and/or prophylaxis of septic shock especially the hypotension associated therewith.

International patent application WO91/04024 (U.S. Patent 5028627) describes the use of an N^{G} substituted arginine or an N^{G}, N^{G}- disubstituted arginine to treat hypotension. In particular. this patent application describes the use of N^{G}-monomethyl-L-arginine (also known as (5)-N⁵[imino(methylamino)methyl]ornithine or L-NMMA) to counteract the production of nitric oxide in induced hypotension and septic shock. Nitric oxide is a potent vasodilator and cytotoxic agent that is normally produced in the endothelium as an endogenous regulator of vascular tone and in macrophages as part of the host defence mechanism. Inappropriate increase in nitric oxide synthesis leads to exaggeration in these actions, so as to cause sustained and pronounced vasodilation, leading to hypofusion of various vital organisms. Furthermore, the substantial increase in the synthesis of nitric oxide in the number of cells leads to cytotoxicity and direct tissue damage, especially to the vascular endothelium.

Kilbourn et al (Proc.Natl.Acad.Sci.U.S.A., 87, 3629, 1990) report the reaction of the flavianate salt of LNMMA with Dowex 1(OH⁻) and titrating the resulting free base of L-NMMA to pH 7.2 with hydrochloric acid. The hydrochloride was not isolated from solution.

We have now found that the hydrochloride of L-NMMA may be obtained as a pure salt, for example in a crystalline form, which has considerable physical advantages, for example in terms of stability. Initial attempts to prepare the hydrochloride in crystalline form gave rise to an amorphous glass which did not crystallise. Crystals of the hydrochloride were unexpectedly obtained when the amorphous glass was left in the presence of ethanol for several months.

Accordingly, the present invention provides the hydrochloride salt of (S)-N⁵[imino(methylamino)methyl]ornithine as a substantially pure salt for example in a solid form and more specifically in a crystalline form. This hydrochloride salt of L-NMMA is anhydrous and not hydroscopic.

Preferably the salt is at least 70%, more preferably at least 90% pure and most preferably greater than 95% pure.

The salt exists in at least three distinguishable isomorphic form(A, Band C) as identified by X-ray diffraction analysis and Differential Scanning Calorimetry (DSC), and the present invention is intended to include each isomorphic form individually or a mixture of two or more isomorphic forms.

Hygroscopicity studies have shown Form A to deliquesce at 65% humidity, whilst Form B is considerably less hygroscopic. DSC shows Form A to be the more thermodynamically stable. DSC has also been used to estimate the melting points of Forms A and B as 219°C and ca.205° respectively.

The present invention also provides a process for preparing crystalline L-NMMA hydrochloride which process comprises reacting L-NMMA with hydrochloric acid and crystallising out the hydrochloride. Preferably the molar ratio of L-NMMA to acid is from 1:1 to 1:5 and, in particular, approximately 1 to 1. The reaction is suitably carried out by dissolving the L-NMMA in a solution of hydrochloric acid (preferably between 0.5 molar and 5 molar and conveniently 2 molar, at a non-extreme temperature, for example between 10° and 80°C, and conveniently at room temperature. The resulting solution is preferably evaporated (for example at a raised temperature, i.e. between 35° and 60°, under reduced pressure). The residue is then crystallised from a suitable solvent, for example by dissolving the residue in a minimum of hot ethanol, conveniently at boiling point, and water. It has been found that seeding the solution containing the hydrochloride salt of L-NMMA assists its crystallisation. Without seeding, the crystallation process may take several months.

The present invention further provides a process for producing L-NMMA hydrochloride which process comprises reacting a salt of L-NMMA other than the hydrochloride with hydrochloric acid and removing the original salt forming ion. Conveniently the reaction is carried out by dissolving the acetate salt of L-NMMA, in aqueous hydrochloric acid, at a concentration between 0.5 and 5 molar conveniently 2 molar. The solution is then evaporated (for example at an elevated temperature, i.e. between 30° and 70°, conveniently 60°, under reduced pressure) The residue is then dissolved in a suitable solvent, for example aqueous ethanol and cooled. Again, seeding the cooled solution with crystals of the hydrochloride salt of L-NMMA assists the crystallisation procedure.

Whilst it may be possible for the hydrochloride of L-NMMA to be administered as the raw chemical, it is preferable to present it as a pharmaceutical formulation. According to a further aspect, the present invention provides a pharmaceutical formulation comprising the hydrochloride of L-NMMA ( the "active ingredient") together with one or more pharmaceutically acceptable carriers therefor and optionally one or more other therapeutic ingredients. The carrier(s) must be "acceptable" in the sense of being compatible with the other ingredients of the formulation and not deleterious to the recipient thereof.

The formulations include those suitable for oral, parenteral (including subcutaneous, intradermal, intramuscular, intravenous and intraarticular), rectal and topical (including dermal, buccal, sublingual and intraocular) administration although the most suitable route may depend upon, for example, the condition and disorder of the recipient. The formulations may conveniently be presented in unit dosage form and may be prepared by any of the methods well known in the art of pharmacy. All methods include the step of bringing into association the active ingredient with the carrier which constitutes one or more accessory ingredients. In general, the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A tablet may be made by compression or moulding, optionally with one or more accessory ingredients. Compressed tables may be prepared by compressing in a suitable machine the active ingredient in a free-flowing form such as a powder or granules, optionally mixed with a binder, lubricant, inert diluent, lubricating, surface active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Formulations for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example, saline, water-for-injection, immediately prior to use or may be stored as a solution ready for injection. Extemporaneous injection solutions and suspensions may be prepared from sterile powders, granules and tablets of the kind previously described.

Formulations for rectal administration may be presented as a suppository with the usual carriers such as coca butter or polyethylene glycol.

Formulations for topical administration in the mouth, for example buccally or sublingually, include lozenges comprising the active ingredient in a flavoured basis such as sucrose and acacia or tragacanth, and pastilles comprising the active ingredient in a base such as gelatin and glycerin or sucrose and acacia.

Preferably the salt will be administered as a solution in water buffered to its own pKa.

Preferred unit dosage formulations are those containing an effective dose, as hereinbelow recited, or an appropriate fraction thereof, of the active ingredient.

It should be understood that in addition to the ingredients particularly mentioned above, the formulations of this invention may include other agents conventional in the art having regard to the type of formulation in question, for example those suitable for oral administration may include flavouring agents.

The compounds of the invention may be administered orally or via injection at a dose of from 1 to 100mg/kg per day and preferably 3 to 50mg/kg per day. Doses of above 3mg/kg per day may preferably be given in a series of smaller doses over a prolonged period, i.e. by infusion over several hours. The dose range for adult humans is generally from 70mg to 7g/day and preferably 200mg to 3.5g/day. Tablets or other forms of presentation provided in discrete units may conveniently contain an amount of compound of the invention which is effective at such dosage or as a multiple of the same, for instance, units containing 70mg to 500mg, usually around 100mg to 300mg.

The compounds of the invention are most suitably administered orally or by injection (intravenous or subcutaneous) and preferably by injection. The precise amount of compound administered to a patient will be the responsibility of the attendant physician. However the dose employed will depend on a number of factors, including the age and sex of the patient, the precise disorder being treated, and its severity. Also the route of administration may vary depending on the condition and its severity.

As mentioned hereinbefore N^{G}-monomethyl-L-arginine hydrochloride may be of use in the treatment and/or prophylaxis of septic shock. Accordingly, in a further aspect of the present invention there is provided N^{G}-monomethyl-L-arginine hydrochloride for use in the manufacture of a medicament for the treatment and/or prophylaxis of septic shock. A yet further aspect of the present invention provides a method of treatment or prophylaxis of septic shock which comprises the administration of a therapeutically effective amount of N^{G}-monomethyl-L-arginine hydrochloride.

The invention will now be described by way of example only.

### Example 1

### Preparation of (S)-N5-[Imino(methylamino)methyl]ornithine hydrochloride (L-NMMA hydrochloride)

A mixture of ornithine hydrochloride (16.86g, 100mMol), N,S-dimethylthiouronium iodide (34.8g, 150mMol) and 2M aqueous sodium hydroxide (100ml) was stirred at 100°C for five hours. The solution was then cooled, adjusted to pH3 with 2M aqueous hydrochloric acid, and applied to a column of Dowex 50W-X8 (H+) resin (200ml wet bed volume). The column was washed with water until the eluate was neutral and then eluted with 0.5M aqueous ammonium hydroxide; fractions of approximately 15ml were taken and were monitored by tlc on silica gel with visualisation by ninydrin. Fractions 26-45 were combined and evaporated at 45°C under reduced pressure to give a colourless resin (9.5g) which was dissolved in 2M aqueous hydrochloric acid and the resulting solution was evaporated at 50°C under reduced pressure. The amorphous residue was treated with hot ethanol (108ml) and the mixture was stirred vigorously, at the boiling point, during the dropwise addition of water (4ml). The residue gradually dissolved and on seeding with a few crystals of the hydrochloride salt began to crystallise. The mixture was cooled and then stood at 4°C for two hours to complete the crystallisation. The product was removed by filtration, washed with ethanol and dried in a vacuum dessicator to give pure L-NMMA hydrochloride (9.6g) as an anhydrous colourless crystalline solid, homogeneous by tlc and hplc and with ¹H nmr and mass spectrum consistent with the proposed structure.

### Example 2

### Preparation of L-NMMA hydrochloride from the acetate monohydrate

L-NMMA acetate monohydrate (186g) was dissolved in 2M aqueous hydrochloric acid (350ml) and the solution was evaporated at 60°C under reduced pressure. The residue was then dissolved in water (ca 150ml) and evaporated under reduced pressure; the redissolution and evaporation process was then repeated twice. The residue was then dissolved in warm water (25ml) with the addition of ethanol (25ml) to aid mobility. The still warm solution was stirred, treated with ethanol (1200ml), seeded with a few crystals and stirring was continued at room temperature for 5 hours. The mixture was kept overnight at 4°C and the crystalline solid was filtered, washed with ethanol and dried in a vacuum desiccator to give pure L-NMMA hydrochloride (108g) identical in all respects with the material described above.

### Example 3

A sample of amorphous hydrochloride, prepared as in Example 1 but without seeding, crystallised after standing under ethanol for about five months at 4°C; this material was used for "seeding" the preparations of Examples 1 and 2.

Samples of seed crystals of L-NMMA hydrochloride are available, on request, from the School of Chemistry, the University of Birmingham, Birmingham B15 2TT.

## Claims

1. N^{G}-monomethyl-L-arginine hydrochloride as a substantially pure salt.

2. N^{G}-monomethyl-L-arginine hydrochloride in a solid form.

3. N^{G}-monomethyl-L-arginine hydrochloride in a crystalline form.

4. The isomorphic form of N^{G}-monomethyl-L-arginine hydrochloride according to Claim 3 which melts at approximately 219°C.

5. The isomorphic form of N^{G}-monomethyl-L-arginine hydrochloride according to Claim 3 which melts at approximately 205°C.

6. A process for preparing crystalline N^{G}-monomethyl-L-arginine hydrochloride which comprises reacting N^{G}-monomethyl-L-arginine with hydrochloric acid and crystallising out the hydrochloride salt.

7. A process according to Claim 6 wherein the ratio of N^{G}-monomethyl-L-arginine to hydrochloric acid is from 1:1 to 1:5.

8. A process for preparing N^{G}-monomethyl-L-arginine hydrochloride which comprises reacting a salt of N^{G}-monomethyl-L-arginine other than the hydrochloride salt with hydrochloric acid and removing the original salt forming ion, followed by crystallising out the hydrochloride salt.

9. A process according to Claims 6, 7 or 8 whereby the crystallisation process is assisted with seeding with one or more crystals of N^{G}-monomethyl-L-arginine hydrochloride.

10. A process for the preparation of a pharmaceutical formulation which comprises the admixture of N^{G}-monomethyl-L-arginine hydrochloride according to Claim 1 with a pharmaceutically acceptable carrier and optionally one or more other therapeutic ingredients.

11. A pharmaceutical formulation comprising N^{G}-monomethyl-L-arginine hydrochloride according to any one of claims 1 to 5 together with one or more pharmaceutically acceptable carriers therefor and optionally one or more other therapeutic ingredients.

## Patentansprüche

1. N^{G}-Monomethyl-L-argininhydrochlorid als ein im wesentlichen reines Salz.

2. N^{G}-Monomethyl-L-argininhydrochlorid in einer festen Form.

3. N^{G}-Monomethyl-L-argininhydrochlorid in einer kristallinen Form.

4. Die isomorphe Form von N^{G}-Monomethyl-L-argininhydrochlorid nach Anspruch 3 mit einem Schmelzpunkt von etwa 219°C.

5. Die isomorphe Form von N^{G}-Monomethyl-L-argininhydrochlorid nach Anspruch 3 mit einem Schmelzpunkt von etwa 205°C.

6. Verfahren zur Herstellung von kristallinem N^{G}-Monomethyl-L-argininhydrochlorid, wobei man N^{G}-Monomethyl-L-arginin mit Salzsäure umsetzt und das Hydrochloridsalz auskristallisiert.

7. Verfahren nach Anspruch 6, wobei das Verhältnis von N^{G}-Monomethyl-L-arginin zu Salzsäure von 1:1 bis 1:5 beträgt.

8. Verfahren zur Herstellung von N^{G}-Monomethyl-L-argininhydrochlorid, wobei man ein N^{G}-Monomethyl-L-argininsalz, ausgenommen das Hydrochloridsalz, mit Salzsäure umsetzt und das ursprüngliche salzbildende Ion entfernt und anschließend das Hydrochloridsalz auskristallisiert.

9. Verfahren nach den Ansprüchen 6, 7 oder 8, wobei der Kristallisationsprozeß durch Animpfen mit einem oder mehreren Kristall(en) von N^{G}-Monomethyl-L-argininhydrochlorid unterstützt wird.

10. Verfahren zur Herstellung einer pharmazeutischen Formulierung, wobei man N^{G}-Monomethyl-L-argininhydrochlorid nach Anspruch 1 mit einem pharmazeutisch verträglichen Träger und ggf. einem oder mehreren therapeutischen Inhaltsstoff(en) vermischt.

11. Pharmazeutische Formulierung, umfassend N^{G}-Monomethyl-L-argininhydrochlorid nach einem der Ansprüche 1 bis 5, zusammen mit einem oder mehreren pharmazeutisch verträglichen Träger(n) dafür und ggf. einem oder mehreren therapeutischen Inhaltsstoff(en).

## Revendications

1. Chlorhydrate de N^{G}-monométhyl-L-arginine, sous forme de sel sensiblement pur.

2. Chlorhydrate de N^{G}-monométhyl-L-arginine, sous forme solide.

3. Chlorhydrate de N^{G}-monométhyl-L-arginine, sous forme cristalline.

4. Forme isomorphique du chlorhydrate de N^{G}-monométhyl-L-arginine, suivant la revendication 3, qui fond à approximativement 219°C.

5. Forme isomorphique du chlorhydrate de N^{G}-monométhyl-L-arginine suivant la revendication 3, qui fond approximativement à 205°C.

6. Procédé de préparation du chlorhydrate de N^{G}-monométhyl-L-arginine cristallin, caractérisé en ce que l'on fait réagir la N^{G}-monométhyl-L-arginine avec de l'acide chlorhydrique et on laisse cristalliser le chlorhydrate sel.

7. Procédé suivant la revendication 6, caractérisé en ce que le rapport de la N^{G}-monométhyl-L-arginine à l'acide chlorhydrique varie de 1:1 à 1:5.

8. Procédé de préparation du chlorhydrate de N^{G}-monométhyl-L-arginine, caractérisé en ce que l'on fait réagir un sel de la N^{G}-monométhyl-L-arginine, autre que le sel de type chlorhydrate, avec de l'acide chlorhydrique et on enlève l'ion formant le sel d'origine, puis on laisse le chlorhydrate se séparer par cristallisation.

9. Procédé suivant la revendication 6, 7 ou 8, caractérisé en ce que le procédé de cristallisation est facilité par ensemencement avec un ou plusieurs cristaux de chlorhydrate de N^{G}-monométhyl-L-arginine.

10. Procédé de préparation d'une composition pharmaceutique, caractérisé en ce que l'on mélange du chlorhydrate de N^{G}-monométhyl-L-arginine suivant la revendication 1 à un véhicule ou excipient pharmaceutique et éventuellement un ou plusieurs autres ingrédients thérapeutiques.

11. Composition pharmaceutique comprenant du chlorhydrate de N^{G}-monométhyl-L-arginine suivant l'une quelconque des revendications 1 à 5, en même temps qu'un ou plusieurs véhicules ou excipients pharmaceutiquement acceptables et éventuellement un ou plusieurs autres ingrédients thérapeutiques.
